(19) Europäisches Patentamt
European Patent Office
Office européen des brevets

(11) Publication number: **0 527 274 A1**

# (12) EUROPEAN PATENT APPLICATION

(21) Application number: **91307271.6**

(22) Date of filing: **08.08.91**

(51) Int. Cl.5: **C08K 5/3492**, C08L 23/02, C08J 3/20, C07D 251/14, B65D 1/00

(43) Date of publication of application: **17.02.93 Bulletin 93/07**

(84) Designated Contracting States: **AT BE CH DE DK ES FR GB IT LI LU NL SE**

(71) Applicant: **DAIKYO GOMU SEIKO LTD.**
**No. 38-2, Sumida 3-chome Sumida-ku**
**Tokyo(JP)**

(72) Inventor: **Sudo, Morihiro**
**32-7, Sumida 3-chome, Sumida-ku**
**Tokyo(JP)**
Inventor: **Muraki, Tomoyasu**
**1-4, Aoyamadai 1-chome**
**Abiko-shi, Chiba(JP)**
Inventor: **Kawachi, Eiji**
**378-3, Sakaeno-machi 5-chome**
**Kiryu-shi, Gunma(JP)**
Inventor: **Kawachi, Yasushi**
**1740-13, Omata-machi**
**Ahikaga-shi, Tochigi(JP)**

(74) Representative: **Allard, Susan Joyce et al**
**BOULT, WADE & TENNANT, 27 Furnival Street**
**London EC4A 1PO (GB)**

(54) **Photo-deterioration inhibitor and composition containing the same.**

(57) A photo-deterioration inhibitor containing a compound represented by the general formula (I):

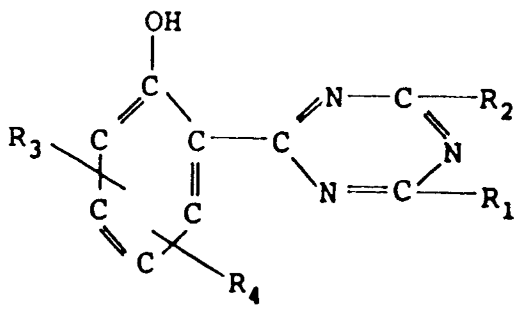

(I)

where $R_1$ and $R_2$ may be same or different and represent hydrogen, chlorine, hydroxyl group, alkoxy group having 1-18 carbon atoms, aryloxy group having 6-8 carbon atoms and mono- or dialkylamino group having 1-4 carbon atoms, respectively, and $R_3$ and $R_4$ may be same or different and represent hydrogen, chlorine, alkyl group having 1-8 carbon atoms, methoxy group and ethoxy group; a photo-deterioration inhibitory resin composition containing a polyolefin resin or polyolefin resin composition and the compound represented by the general formula (I); and a container formed from the photo-deterioration inhibitory resin composition containing the polyolefin resin or polyolefin resin composition and the compound represented by the general formula (I).

EP 0 527 274 A1

The present invention relates to a novel photo-deterioration inhibitor, a photo-deterioration inhibitory resin composition or a light-resisting resin composition containing the photo-deterioration inhibitor and to a photo-deterioration inhibitory container or a light-resisting container formed from the photo-deterioration inhibitory resin composition.

It is well known that the olefin resin is so unstable to light, oxygen and heat as to cause deterioration, discoloration and reduction in mechanical strength in a short period of time to be incapable of being practically used. It is a common practice for retarding the above phenomenon to use deterioration inhibitors such as aging inhibitor, ultraviolet light absorbing agent, light screen, metal damage inhibitor and the like as additives.

However, use of the deterioration inhibitors in the prior art all causes unsatisfactory effect, discoloration and oxidation, resulting in making mechanical properties so poor as not to be practically used.

That is, amine compounds such as N-phenyl-N'-isopropyl-p-phenylenediamine, 2,2,4-trimethyl-1,2-dihydroquinoline polymer, diphenylamine•acetone condensate and the like, which are known as aging inhibitors to inhibit deterioration of resin due to oxygen and heat, show good aging inhibiting effects, but their application is confined to particular products because of coloring, discoloration, toxicity and the like.

The deterioration inhibitors used in colorless or light-colored products may generally include phenolic compounds, phosphorous compounds, sulfur-containing phenolic compounds and the like. Examples of the phenolic compound may include 2,6-di-t-butyl-4-methylphenol (BHT), 2,6-di-t-butyl-anisole (BHA), 4,4'-butylidene-bis(3-methyl-6-t-butylphenyl) (trade name, Antage W 300, marketed by Kawaguchi Chemical Industrial Co., Ltd.), octadecyl-3(4'-hydroxy-3',5'-di-t-buthylphenyl)propionate (trade name, Irganox 1076, marketed by Ciba Geigy A.G.), tetrakis[methylene(3,5-di-t-butyl-4-hydroxyphenyl)propionate]methane (trade name, Irganox 1010, marketed by Ciba Geigy A.G.), Tocopherol, 1,1,3-tris(2-methyl-4-hydroxy-5-t-butylphenyl)butane (trade name, Topanol CA, marketed by Imperial Chemical Industries Ltd.), 1,3,5-trimethyl-2,4,6-tris(3,5-di-t-butyl-4-hydroxybenzyl)benzene (trade name, Ionox 330, marketed by shell Chemical Co., Ltd.) and the like. Examples of the phosphorous compound may include tetranonylphenyl phosphite (trade name, Antage TNP, Kawaguchi Chemical Industrial Co., Ltd.) and the like. Examples of the sulfur-containing phenolic compound may include distearyl thiodipropionate (trade name, Antigene, TPS, marketed by Sumitomo Chemical Co., Ltd.), 4,4'-thiobis(6-t-butyl-3-methylphenol) (trade name, Antage RC, marketed by Kawaguchi Chemical Co., Ltd.) and the like. The above deterioration inhibitors, however, show poor aging inhibiting effect particularly on ultraviolet light.

Examples of the aging inhibitor to light, particularly ultraviolet light may include benzophenone compounds such as 2-hydroxy-4-methoxybenzophenone (trade name, Sumisorp 110, marketed by Sumitomo Chemical Co., Ltd.), 2,4-dihydroxy-4-methoxybenzophenone (trade name, Sumisorp 100, marketed by Sumitomo Chemical Co., Ltd.), 2-hydroxy-4-n-octoxybenzophenone (trade name, Sumisorp 130, marketed by Sumitomo Chemical Co., Ltd.; trade name, Biosorp 130, marketed by Kyodo Pharmaceutical Co., Ltd.) and the like; benzotriazole compounds such as 2-(2'-hydroxy-5'-methylphenyl)benzotriazole (trade name, Tinuvin P, marketed by Ciba Geigy A.G.), 2-(2'-hydroxy-3',5'-di-t-pentylphenyl)benzotriazole (trade name, Tinuvin 622 LD, marketed by Ciba Geigy A.G.), 2-(2'-hydroxy-3'-t-butyl-5-methylphenyl)-5-chloro-benzotriazole (trade name, Tinuvin 326, marketed by Ciba Geigy A.G.), 2(2'-hydroxy-4-n-octoxyphenyl)-benzotriazole (trade name, Tinuvin 510, marketed by Ciba Geigy A.G.) and the like; aryl ester compounds such as p-octylphenyl salicylate (trade name, Seasorp 203, marketed by Shiraishi Calcium Kaisha Ltd.) and like.

Examples of commercially available light screens may include hindered amine compounds such as bis-(2,2,6,6-tetramethylpipezyl sebacate (trade name, Sanol LS-770, marketed by Sankyo Co., Ltd.; trade name, Tinuvin 770, marketed by Ciba Geigy A.G.), 1,3,8-triaza-7,7,9,9-tetramethyl-3-n-octyl-spiro[4,5]decane-2,4-dion (trade name, Sanol LS-772, marketed by Sankyo Co., Ltd.) and the like; nickel salts such as bis-(diisopropyl-dithiocarbamate)nickel salt, bis(di-n-butyl-dithiocarbamate) nickel salt (trade name, NBC, marketed by Du Pont de Nemours & Co., marketed by Kawaguchi Chemical Co., Ltd.), bis(2-hydroxy-5-methoxyphenyl-N-n-butylamine) nickel salt, and the like.

The benzophenone compound has such a broad absorptivity as to show high coloring properties. The benzotriazole compound has a narrow ultraviolet wave length range and shows no absorption in the visible light range, result in showing initial coloring but a relatively stable tint. The aryl ester compound is colored to yellow. The hindered amine compound (hereinafter is referred to as HALS) provides high photo-deterioration inhibiting effect and low coloring properties, but is unable to be used for acidic resins such as polycarbonate resin, polyester resin, vinyl chloride resin, vinylidene chloride resin and the like because of its basic properties. It also shows bitter taste and reacts with polyurethane, resulting providing little effect. The nickel salt is confined to special application, because it makes the product light green and slows toxicity.

Moreover, the above commercially available compounds has poor compatibility and diffusing properties with the resin, and may cause blooming and whitening, resulting in that their application to transparent products are restricted with little effect.

Although it has recently been demanded to keep contents kept in a container or bag formed from the above resins at high purity for a long period of time, the present situation thereof is as above mentioned and studies and researches on the above demand are unsatisfactory.

Thus, the resin container for use in medicines requires particularly light-shielding properties. For example, a wrapping cloth of a drug ampul bottle containing vitamin $K_1$ compound, vitamin $K_2$ compound, an injection for use in nervous disease treatment, etc. is wrapped with a red or orange film or aluminum film to shield light for inhibiting deterioration (see Packing Technology, 1896, No. 12). There is also proposed a polyethylene (hereinafter referred to as PE) container having a multi-laminated structure, in which a light screen such as carbon black, titanium, aluminum, pigment or the like is added to a part of the inner layers (see Japanese Patent Application Laid-Open No. 39444/87). As above mentioned, none of the resins, to which photo-deterioration inhibitors and additive were added in order to impart light resisting properties, were satisfactory. However, at present, it has been demanded to keep the contents of the resin containers at high purity for a long period of time safely without influence of light thereon.

It is an object of the present invention to provide a novel photo-deterioration inhibitor overcoming the disadvantages of those in the prior art.

It is another object of the present invention to provide an olefin resin containing a novel photo-deterioration inhibitor and having improved light resisting properties.

It is another object of the present invention to provide a light resistant container formed from an olefin resin composition and capable of preserving the contents therein safely for a long period of time without photo-deterioration.

That is, the present invention provides a photo-deterioration inhibitor containing a compound represented by the general formula (I):

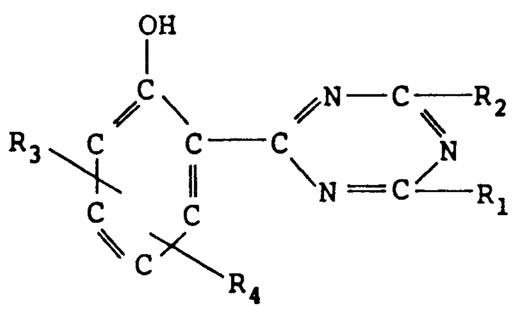

(I)

where $R_1$ and $R_2$ may be same or different and represent hydrogen, chlorine, hydroxyl group, alkoxy group having 1-18 carbon atoms, aryloxy group having 6-8 carbon atoms and mono- or diakylamino group having 1-4 carbon atoms respectively, and $R_3$ and $R_4$ may be same or different and represent hydrogen, chlorine, alkyl group having 1-8 carbon atoms, methoxy group and ethoxy group.

The present invention further provides a photo-deterioration inhibitory resin composition containing a polyolefin resin or polyolefin resin composition and the compound represented by the general formula (I), and provides a container formed from the photo-deterioration inhibitory resin composition containing the polyolefin resin or polyolefin resin composition and the compound represented by the general formula (I).

The present inventors made extensive studies to find that the photo-deterioration inhibitor containing hydroxyaryl-S-triazine compounds (hereinafter may be referred to as HAFT) represented by the general formula (I) are very effective, that the resin composition containing HAFT as the additive shows high light resisting properties, and that the container formed from the resin composition containing HAFT as the additive is capable of protecting the container itself as well as the contents therein against deterioration due to light.

With reference to the drawing:

Fig. 1 shows absorption spectra of HAFT compounds, i.e. compound Nos. 14 and 15 in the present invention, in which UV-A means a long wave length ultra-violet light, UV-B means a medium wave length ultra-violet light and UV-C means a short wave length ultra-violet light.

Explanation is first made one the hydroxyaryl-S-triazine compound represented by the general formula (I) in the present invention.

Specific examples of HAFT in the present invention may include the following compounds:

Compound No. 1: 2,6-dichloro-4-(2-hydroxyphenyl)-S-triazine

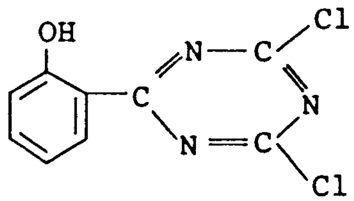

Compound No. 2: 2-chloro-6-methoxy-4-(2-hydroxyphenyl)-S-triazine

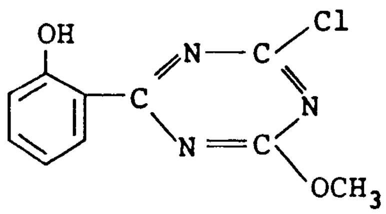

Compound No. 3: 2,6-diphenoxy-4-(2-hydroxyphenyl)-S-triazine

melting point: 164-165 °C

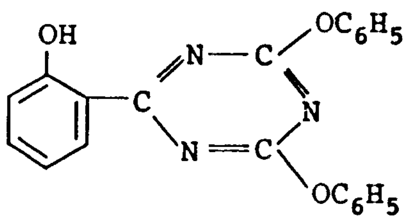

Compound No. 4: 2,6-di(methylphenoxy)-4-(2-hydroxymethylphenyl)-S-triazine

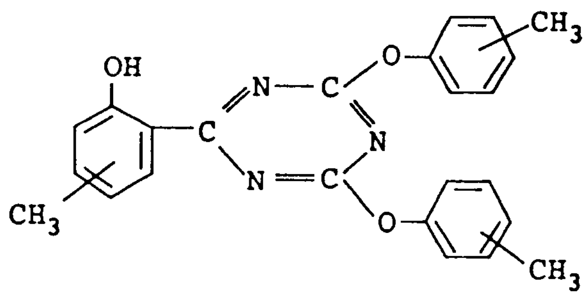

Compound No.5 : 2,6-dimethyoxy-4-(2-hydroxy-methyl-t-butylphenyl)-S-triazine

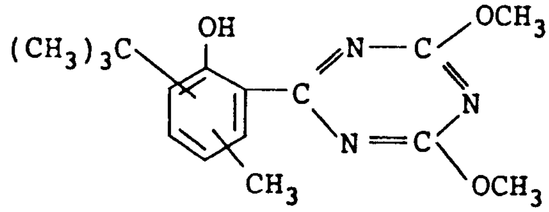

Compound No. 6: 2,6-di(dimethylamino)-4-(2-hydroxy-3,5-dimethylphenyl)-S-triazine

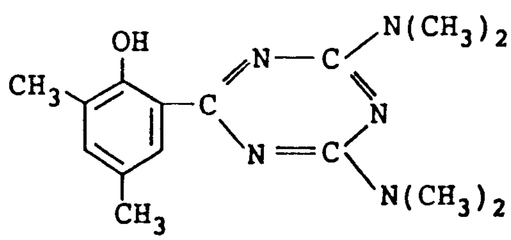

Compound No. 7: 2,6-di(dimethylphenoxy)-4-(2-hydroxyoctylphenyl)-S-triazine

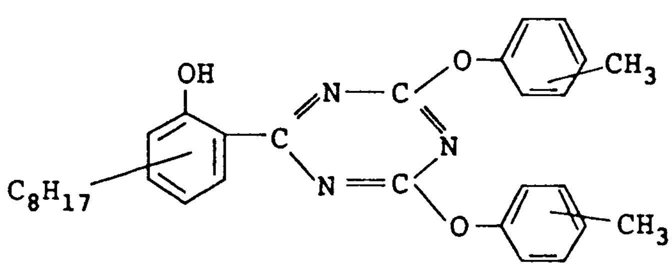

Compound No. 8: 2,6-di(methoxy)-4-(2-hydroxy-5-methylphenyl)-S-triazine

melting point: 134-135 ° C
UV $\lambda_{max}$ 268, 347 nm

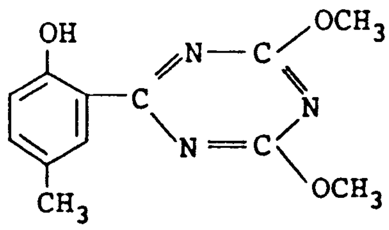

Compound No. 9: 2,6-dilauryloxy-4-(2-hydroxy-di-t-butylphenyl)-S-triazine

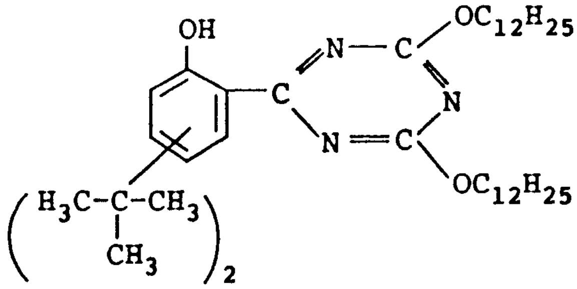

Compound No. 10: 2-methoxy-6-phenoxy-4-(2-hydroxyphenyl)-S-triazine

melting point: 150-151 ° C

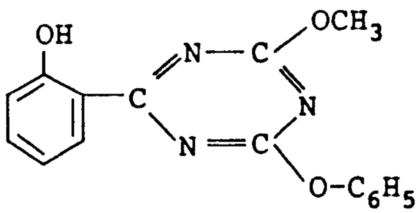

Compound No. 11: 2-p-methylphenoxy-6-methoxy-4-(2-hydroxyphenyl)-S-triazine

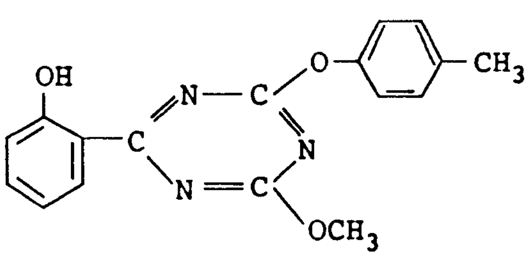

Compound No. 12: 2-dimethylamino-6-methoxy-4-(2-hydroxy-5-methylphenyl)-S-triazine

melting point: 137-138 ° C
UV $\lambda_{max}$ 262 nm

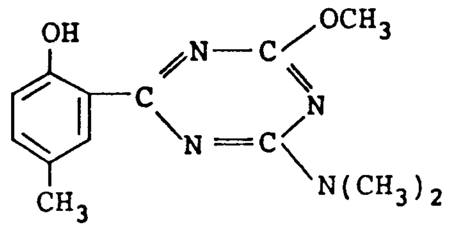

Compound No. 13: 2,6-bis(dimethylamino)-4-(2-hydroxy-5-methylphenyl)-S-triazine

melting point: 162-163 ° C
UV $\lambda_{max}$ 256 nm

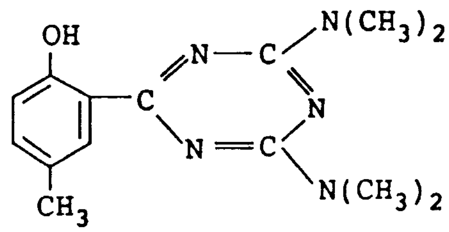

Compound No. 14: 2,6-dimethoxy-4-(2-hydroxyphenyl)-S-triazine

melting point: 112-113 ° C
UV $\lambda_{max}$ 267 nm

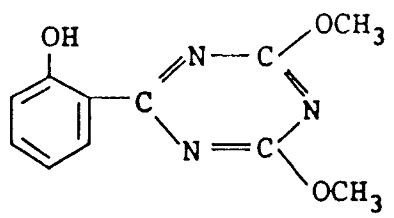

Compound No. 15: 2,6-dimethoxy-4-(2-hydroxy-5-methoxyphenyl)-S-triazine

melting point: 135-136 ° C
UV $\lambda_{max}$ 268 nm

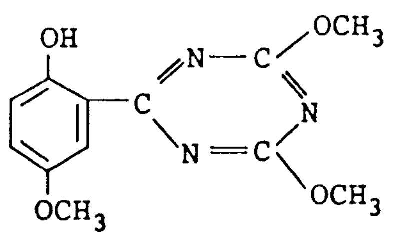

Compound No. 16: 2,6-dimethoxy-4-(2-hydroxy-5-chlorophenyl)-S-triazine

melting point: 145-146 ° C
UV $\lambda_{max}$ 350 nm

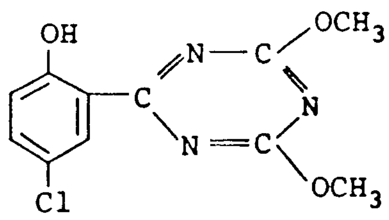

Compound No. 17: 2[p-methylphenyl]-4-(2-hydroxy-5-methylphenyl)-S-triazine

melting point: 216-217 ° C
UV $\lambda_{max}$ 237 nm

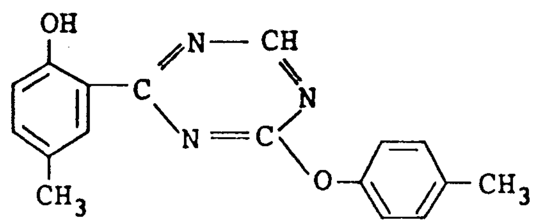

Compound No. 18: 2,6-dihydroxy-4-(2-hydroxyphenyl)-S-triazine

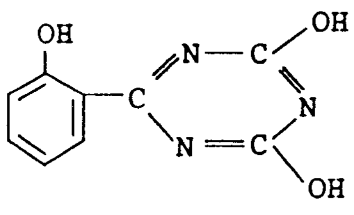

Compound No. 19: 2-phenoxy-6-hydroxy-4-(2-hydroxyphenyl)-S-triazine

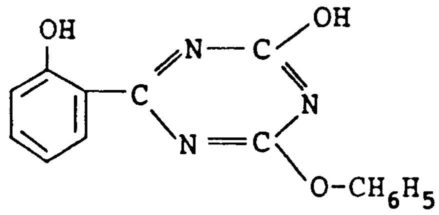

compound No. 20: 2,6-bis(dimethylamino)-4-(2-hydroxyphenyl)-S-triazine

melting point: 145-146 ° C
UV $\lambda_{max}$ 256 nm

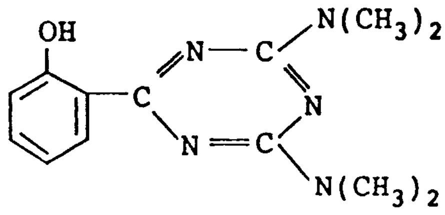

Compound No. 21: 2-phenoxy-6-di(methylamino)-4-(2-hydroxyphenyl)-S-triazine

melting point: 147-148 ° C

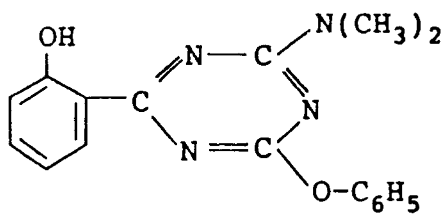

Compound No. 22: 2-methoxy-6-(p-tolyloxy)-4-(2-hydroxy-5-methylphenyl)-S-triazine

melting point: 131-132 ° C
UV $\lambda_{max}$ 271 nm

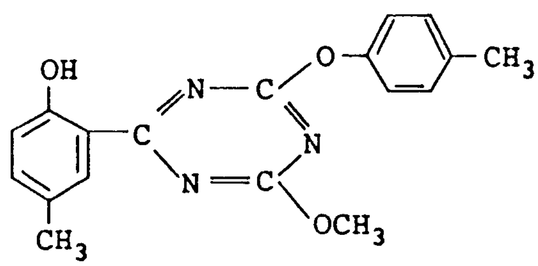

Compound No. 23: 2,6-bis(tolyloxy)-4-(2-hydroxy-5-mexhylphenyl)-S-triazine

melting point: 158-159 ° C

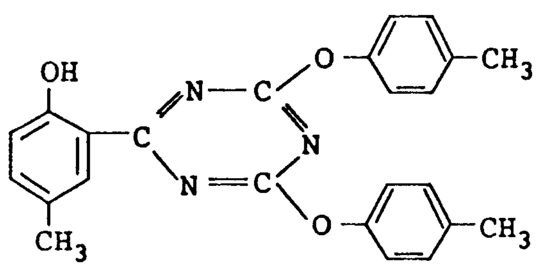

The compound No. 1, i.e. 2,6-dichloro-4-(2-hydroxyphenyl)-S-triazine as an example of HAFT compounds in the present invention is prepared by a process which comprises heating a mixture of cyanuryl chloride ($C_3Cl_3N_3$) and phenol in a solvent such as methanol refluxing methanol to carry out dihydrochlorination, followed by irradiating ultraviolet light by use of a high pressure mercury lamp to cause to take place rearrangement reaction to ortho position. Presence of an unreacted starting material on the rearrangement reaction makes aging inhibiting properties poor. The arrangement reaction takes place not only at ortho-position but also at para-position. The latter case results less effect compared with the former cede.

The photo-deterioration inhibitor of the present invention contains the HAFT compound represented by the general formula (I) as the essential component, but may contain the aging inhibitor, ultraviolet light absorbing agent and ultraviolet light shielding agent, which results in improving photo-deterioration inhibitory properties and light resisting properties.

Preferable examples of the aging inhibitor to be used in combination may include BHT, BHA, Antage W-300 (trade name, Kawaguchi Chemical Industrial Co., Ltd.), Irganox 1076 (trade name, marketed by Ciba Geigy A.G.), Irganox 1010 (trade name, marketed by Ciba Geigy A.G.), Topanol CA (trade name, marketed by Imperial Chemical Industries Ltd.), MARKAD-330 (trade name, marketed by Adeka Argus Chemical Co., Ltd.), Irganox 1330 (trade name, marketed by Ciba Geigy A.G.), 3,9-bis[1,1-dimethyl-2-{β-3-t-butyl-4-hydroxy-5-methylphenyl)propionyloxy}ethyl]-2,4,8,10-tetraoxaspiro[5,5]undecane (trade name, Sumiliser GA-80, marketed by Sumitomo Chemical Co., Ltd.; trade name, MARKAD-80, marketed by Adeka Argus Chemical Co., Ltd.), triethylene glycol bis[3-(3-t-butyl-5-methyl-4-hydroxyphenyl)]propionate (trade name, Irganox 245, marketed by Ciba Geigy A.G.), tocophenol, tris-(3,5-di-t-butyl-4-hydroxybenzyl)isocyanurate (trade name, MARKAD-20, marketed by Adeka Argus Chemical Co., Ltd.), di(2,4-di-t-butylphenyl)-pentaerythritol diphosphite (trade name, MARKPEP-24G, marketed by Adeka Argus Chemical Co., Ltd.), distearyl pentaerythritol diphosphite (trade name, MARKPEP 8, marketed by Adeka Argus Chemical Co., Ltd.), Antage TNT (trade name, marketed by Kawaguchi Chemical Industrial Co., Ltd.), tris(2,4-di-t-butylphenyl)phosphite (trade name, MARK2112, marketed by Adeka Argus Chemical Co., Ltd.), tetrakis(2,4-di-t-butylphenyl) 4,4'-biphenylene phosphite (trade name, Irgafos P-EPQ-FF, marketed by Imperial Chemical Industries Ltd.), Sumilizer TPS (trade name, Sumitomo Chemical Co., Ltd.), pentaerythritol tetrakis-(β-lauryl-thiopropionate) (trade name, Sumilizer-TP-D, marketed by Sumitomo Chemical Co., Ltd.), Antage RC (trade name, Kawaguchi Chemical Industrial Co., Ltd.), and the like. The amount of the aging inhibitor used in combination is preferably in the range of 0.05 to 2 parts by weight per unit part by weight of the olefin resin.

Preferable examples of the ultraviolet light absorbing agent used in combination may include 2-(3,5-di-t-amyl-2-hydroxyphenyl)benzotriazole (marketed by Ciba Geigy A.G.), 2-ethoxy-2'-ethyl oxalic acid anilide (marketed by Ciba Geigy A.G.), 2,4'-dihydroxy-4,4'-dimethoxybenzophenone (trade name, Chemisorp 1011, marketed by Chemipro Kasei Kaisha Ltd.), 2-hydroxy-di-4-n-octoxybenzophenone (trade name, Biosorp 130, marketed by Kyodo Pharmaceutical Co., Ltd.), Sumisorp 110 (trade name, marketed by Sumitomo Chemical Co., Ltd.), Sumisorp 100 (trade name, marketed by Sumitomo Chemical Co., Ltd.), Tinuvin P, Tinuvin P622LD, Tinuvin P326, Tinuvin P510 (trade names, marketed by Ciba Geigy A.G. respectively), Seasorp 203 (trade name), 3,5-di-t-butyl-4-hydroxybenzoate (trade name, Chemisorp 112, marketed by Chemipro Kasei Kaisha Ltd.), dimethyl succinate-1-(2-hydroxyethyl)-4-hydroxy-2,2,6,6-tetramethylperidine polycondensate (marketed by Ciba Geigy A.G.), 2-(3,5-di-t-butyl-4-hydroxybenzyl)-2-n-butyl malonic acid bis(1,2,6,6-pentamethyl-4-piperidine) (trade name, Tinuvin 144, marketed by Ciba Geigy A.G.), Sanol LS-770, Sanol LS-772 (trade names, marketed by Sankyo Co., Ltd. respectively), and the like. The amount of the ultraviolet light absorbing agent to be used in combination is preferably in the range of 0.1 to 2 parts by weight per 100 parts by weight of the olefin resin.

Preferable examples of the ultraviolet light shielding agent used in combination may include titanium oxide, finely powdered titanium oxide, surface treated titanium oxide, finely powdered zinc oxide, surface treated zinc oxide, and the like. The amount of the ultraviolet light shielding agent to be used in combination is preferably in the range of 0.001 to 2 parts by weight per 100 parts by weight of the olefin resin.

Addition of the photo-deterioration inhibitor containing as the major component the HAFT compound of the present invention to the polyolefin resin composition makes it possible to obtain a light-resisting resin composition having an improved resistance to photo-deterioration. The above aging inhibitor, ultraviolet light absorbing agent and ultraviolet light shielding agent used in combination as above mentioned, may also be used in combination.

Examples of the polyolefin resin used in the resin composition of the present invention may include homopolymers of polyethylenes such as low-density polyethylene, medium-density polyethylene and high-density polyethylene, polypropylene, polybutadiene, dicyclopentadiene and hydrogenated product of its polymer, polyvinylidene chloride, polybutadiene and the like; and copolymers of ethylene-propylene, ethylene-terephthalate, polyurethane, styrene-butadiene, acrylo-nitrile-styrene-butadiene, propylene-butene, nylon, ethylene-butene, ethylene-vinylidene acetate, and the like; mixtures thereof, polymer alloys thereof, and the like.

The polyolefin resin used in the present invention may also include ones crosslinked with an organic peroxide, ones radiation-crosslinked or electron radiation-crosslinked by use of electron radiation or electromagnetic wave, etc., and ones subjected to grafting with maleic acid, maleic anhydride, etc.

The HAFT content in the resin composition is in the range of 0.1 to 10 parts by weight. In the case where other additives are added, the aging inhibitor content is in the range of 0.05 to 2 parts by weight, the ultraviolet light absorbing agent content is in the range of 0.1 to 2 parts by weight, and the ultraviolet light shielding agent content is in the range of 0.01 to 2 parts by weight based on the total amount of the resin composition, respectively.

The container capable of inhibiting photo-deterioration may be obtained by molding from the resin composition of the present invention. The container is capable of sufficiently inhibiting photo-deterioration not only of the container itself but also of liquid or solid contents contained therein, and is an improved container which is capable of safely preserving the contents for a long period of time without causing deterioration, discoloration, etc.

The container may be prepared by a known molding process to use the above polyolefin resin composition as the major starting material, for example, by slow molding etc. For example, a container may be prepared by a process which comprises mixing the HAFT compound and, if needed, other additives with a starting resin such as polyolefin resin, further mixing by means of a mixing means such as pressure kneader, Banbury mixer or the like, and single- or double-screw extruder, allowing the resin to be heat melted, followed by injection molding or T blow molding to mold to a shape of a container.

The HAFT compound contained as the major component in the photo-deterioration inhibitor of the present invention is capable of inhibiting deterioration of the polyolefin resin composition due to light, oxygen, heat, etc. because of the presence of hydroxyl group in its phenol structure. This may be because of tautomerism fast in an excited state between three carbons and nitrogen, hydrogen and oxygen particularly to light and ultraviolet light, and the absorbing or shielding wave length is not only of a strong one but also reaches a long wave length, i.e., long wave length ultraviolet light = UV-A (see Fig. 1). The above phenomenon is somewhat different from that of the compounds known as aging inhibitor and light screen in the art. Therefore, the HAFT compound of the present invention is capable of absorbing and shielding not only ultraviolet light but also those in long or high wave length region.

Since the HAFT compound of the present invention has good compatibility and diffusing properties with the resin or the resin composition, it may be added in a large amount to the resin for improving photo-deterioration inhibiting effect. The above compound of the present invention may preferably be added in an amount of 0.1 to 10 parts by weight. Even if it is added in a large amount, neither blooming takes place on the resin surface, nor crystallization to make transparency poor takes place in the interior of the resin. Since addition of the compound of the present invention in such a large amount as above does not cause to make the resin opaque, the compound of the present invention shows photo-deterioration inhibiting properties, resulting in providing a transparent container. The transparent container having the above light-shielding properties has such advantage that discoloration of the content such as chemicals contained therein, presence of impurities, etc. may be found from the outside at a glance.

The use of the compound only of the present invention provides satisfactory photo-deterioration inhibiting effects, but the wave length absorbing effect of the HAFT compound of the present invention is somewhat different from those of the compounds in the art. Such being the case, a combined use of the above compound of the present invention with the conventional aging inhibitor such as phenolic compounds, phosphorous compounds and sulfur-containing phenolic compounds, ultraviolet light absorbing agent and ultraviolet light shielding agent makes it possible to provide preferable synergistic effect, i.e., improved inhibiting effect on deterioration due to light, heat, oxygen, metal, humidity, etc.

The addition of the ultraviolet light absorbing agent in the art resulted in being colored yellow and yellowish brown, but the use of the photo-deterioration inhibitor of the present invention does not result in coloring.

## Example

The present invention is explained more in detail with reference to the following examples, but it is to be understood that the scope of the invention is not to be limited to the examples described herein.

Example 1

Synthesis of 2,6-dimethoxy-4-(2-hydroxy-5-methyl)-S-triazine (Compound No. 8):

1) To 180 ml of methanol is added 60.6 g of sodium carbonate to be dissolved. The resulting solution is kept at about 30 ° C, followed by dropping 56 g of cyanuryl trichloride ($C_3Cl_3N_3$) with agitation, and stirring for 5 hours to be reacted. Thereafter, the reaction mixture is poured into 200 g of ice, followed by crystallizing to be separated, and recrystallizing with petroleum ether to obtain 2,4-dimethoxy-2-chloro-S-triazine having a melting point of 65-70 ° C.
2) In 300 ml of chloroform is dissolved 52 g of 2,6-dimethoxy-4-chloro-S-triazine obtained as above, followed by dropping with agitation at about 60 ° C a mixture of 35 g of p-cresol, 15 g of sodium hydroxide and 100 ml of water, and reacting for 10 hours with agitation. After the completion of the reaction, the reaction mixture is thoroughly washed a 2 wt% aqueous sodium hydroxide solution, followed by drying to obtain 2,4-dimethoxy-6-(4-methylphenoxy)-S-triazine at a yield of about 92%.
3) In ethanol is dissolved 2,6-dimethoxy-4-(4-methylphenoxy)-S-triazine so as to form a 10 wt% solution, the solution is then irradiated with a high pressure mercury lamp (a mercury lamp containing ozone and having a major wave length of 240-320 nm, marketed by Hitachi Denki Ltd.) with agitation to rearrange hydroxyl group of the above compound to o-position for being crystallized. The crystals thus obtained is recrystallized with ligroin. Properties of the crystals thus obtained are shown as follows.
Melting point: 134-135 ° C
Infrared absorption (KBr disc):
OH group absorption at ~2760 $cm^{-1}$

| Elemental analysis (theoretical values given in parentheses): | |
|---|---|
| carbon | 58.68 (58.29) |
| nitrogen | 17.08 (17.00) |
| hydrogen | 5.57 (5.30) |
| oxygen | balance |

The qualitative test results of an intermediate compound, i.e., 2,6-dimethoxy-4-(4-methylphenoxy)-S-triazine obtained in the above step 1) and of a final compound, i.e., crystals obtained in the above step 3) are shown in the following Table-1. It was found from the above results that 2,6-dimethoxy-4-(2-hydroxy-5-methyl)-S-triazine as Compound No. 8 was synthesized. Respective test were carried out as follows.

① Melting point: It was determined in accordance with the melting point measuring method, general testing method 40, the 10th Revisionary Japanese Pharmacopoeia.

② $UV_{max}$ value (nm): It was determined in accordance with absorbance measuring method as in the case of the above ①. It shows a maximum wave length $\lambda_{max}$ nm.

③ Identification of the phenolic hydroxyl group: About 30 mg or one drop, when liquid, of a sample is dissolved in a solvent such as water, ethanol, chloroform or the like, followed by adding 2 to 3 drops of the above solvent. To the resulting sample are added one drop of pyridine and one drop of a solution separately prepared by dissolving 1 g of ferric chloride in 20 ml of methanol to observe a color reaction. An identification limit of the phenolic hydroxyl group is 1 $\mu$g.

④ Identification of phenolic hydroxyl group: An ethyl alcohol solution containing 0.1 g of a sample is prepared, followed by adding 2 to 3 drops of a suspension of 2,6-dichloroquinonechloroimide to observe a color reaction to a deep blue color.

Table-1

| Items | Intermediate [product obtained at step 2)] | Final product (HAFT of the present invention |
|---|---|---|
| Formula | -O- | -OH (o-isomer) |
| m.p. (°C) ① | 89~90.5 | 134~135 |
| $UV_{max}$ (nm) ② | | |
| (Ethanol solution) | very small | 265, 331 |
| (Cyclohexane solution) | very small | 268, 347 |
| Color reaction | | |
| Ferric chloride ③ | none | red |
| 2,6-dichoroquinone-chloroimide ④ | none | deep blue |

Example 2

Synthesis of 2,6-bis(dimethylamino)-4-(2-hydroxyphenyl)-S-triazine (Compound No. 20):

1) Synthesis of 2,6-di-(dimethylamino)-4-phenoxy-S-triazine:

To 58 g of phenol is added 100 g of methanol, followed by adding 29 g of anhydrous sodium carbonate, equipping a reflux condensed and a stirrer to heat for about 10 minutes, cooling down to about 5°C, slowly adding with agitation a mixed solution of 30.6 g of cyanuryl trichloride and 30.6 g of methanol, and reacting for about 5 hours at about 30°C, adding 60 g of dimethylamine to react for 10 hours at about 70°C, evaporating methanol, flowing out into iced water to be crystallized, and recrystallizing with ligroin. The product had a melting point of 103-106°C.

2) Synthesis of 2,6-dimethylamine(dimethylamino)-4-(2-hydroxyphenyl)-S-triazine:

A 10 wt% ethanol solution of 2,6-di-(dimethylamino)-4-phenoxy-S-triazine obtained at the above step 1) is formed, followed by irradiating for about 10 hours by use of a low pressure mercury lamp with agitation at room temperature for taking place an intramolecular rearrangement to synthesize 2,6-dimethylamine-(dimethylamino)-4-(2-hydroxyphenyl)-S-triazine, and recrystallizing with ligroin. Properties of the crystals thus obtained are shown as follows.

Melting point: 145-146°C

UV $\lambda_{max}$ : 256 nm

| Elemental analysis (theoretical values given in parentheses): | |
|---|---|
| carbon<br>nitrogen<br>hydrogen<br>oxygen | 60.48 (60.22)<br><br>6.92 (6.60)<br>balance |

Compound Nos. 1-7, 9-19 and 21-23 of the present invention may be synthesized in the same manner as above.

Example 3

To a low density polyethylene free of aging inhibitor are added to the HAFT compounds of the present invention and commercially available additives as shown in the following Table-2, followed by mixing in Henschel mixer at 100-110 °C, subjecting to injection molding for extruding in the form of a film having a thickness of 0.3 mm. The plate was subjected to the following tests. Test results are also shown in Table-2.

⑤ Photo-deterioration test:

Irradiation of ultraviolet light having a major wave length of 340-450 nm was carried out onto the film by use of Sunshine Weather Meter (marketed by Suga Test Instrument Co., Ltd., trade name: WF-SUN-Hc) to determine degree of discoloration and an irradiation time, in which breaking extension should half of that of a non-irradiated film and to determine a degree of deterioration due to ultraviolet light. (The breaking extension of the non-irradiated film is about 150%. The test results shown in Table-2 have correlationship with a time, in which a weight loss in the thermogravimetric analysis shows 50% by weight with increase in temperature, and this test may also be referred to as T-50 curve.)

⑥ A film having a thickness of 0.05±0.01 mm is placed in a constant temperature bath under normal pressure of oxygen, followed by raising temperature to determine an aging speed at 180±0.2 °C due to absorption of oxygen, and determining a time, in which the absorbing speed was abruptly increased, by use of a tester marketed by Toyo Seiki Seisakusho Co., Ltd. (From the above test, it may be said that an aging inhibitor showing a low oxygen absorbing speed has high resistance to oxidation and high thermal resistance.)

Table-2

| Examples | Compounds of the present invention and additives | Amount (phr) | Photo-deterioration test ⑤ | Oxygen absorption test ⑥ | Notes |
|---|---|---|---|---|---|
| Example 1 | HAFT No. 2 | 0.2 | 10.5 | 3 | |
| Example 2 | " No. 5 | 0.2 | 15 | 6 | |
| Example 3 | " No. 6 | 0.2 | 13.5 | 10.5 | |
| Example 4 | " No. 8 | 0.2 | 18 | 6 | |
| Example 5 | " No. 9 | 0.2 | 9 | 4.2 | |
| Example 6 | " No. 9 | 10 | 30 | 10 | transparent product |
| Example 7 | " No. 18 | 0.2 | 7.5 | 4.3 | |
| Example 8 | " No. 19 | 0.2 | 6.5 | 2.0 | |
| Comparative Example 1 | none | - | 1.0 | 2 | |
| Comparative Example 2 | titanium oxide (finely powdered) | 0.3 | 10 | 1.5 | whitening, light screen |
| Comparative Example 3 | Antage W300 | 0.2 | 6.0 | 9 | antioxidant |

Table-2 (continued)

| Examples | Compounds of the present invention and additives | Amount (phr) | Photo-deterioration test ⑤ | Oxygen absorption test ⑥ | Notes |
|---|---|---|---|---|---|
| Comparative Example 4 | Sumisorb 130 | 0.2 | 9.0 | 3 | ultraviolet light absorbing agent |
| Comparative Example 5 | Tinuvin P | 0.2 | 7.0 | 3.5 | ultraviolet light absorbing agent |
| Comparative Example 6 | Sanol SL-770 | 0.2 | 11.0 | 4.5 | light screen |

As shown in Table-2, the polyethylene film containing the HAFT compounds in the present invention show no discoloration due to irradiation of ultraviolet light, slow change in extension properties and good transparency. Addition of 1 phr (Example 6) of HAFT resulted in slowing down the speed of change in properties. On the other hand, even if the compound of the present invention, the compound (Example 8) having a phenoxy structure shows high resistance to ultraviolet light irradiation, but low resistance to oxygen

aging.

Example 4

Respective additives shown in Table-3 were added onto the polyethylene terephthalate obtained by heating and polycondensating dimethylene ester of terephthalic acid and ethylene glycol in the presence of a catalyst, hereinafter referred to as PET, in the form of polyester•polyester type, marketed by Toyobo Co., Ltd. under a trade name of Pelprene S-6001), followed by mixing and melting at 250±10 ° C in an extruder, molding at 240 ° C under a molding pressure of 25 $kg/cm^2$, forming in the form of a film having a thickness of 0.2 mm. The film was subjected to the photo-deterioration test in the same manner as in Example 3. The results are shown in Table-3.

Table-3

| Examples | Formulations | Amount (phr) | Photo-deterioration test (hr) ⑤ | Notes |
|---|---|---|---|---|
| Example 9 | HAFT No. 8 of the present invention | 0.4 | 30 | |
| Example 10 | HAFT No. 8 of the present invention | 0.4 | | |
| | titanium oxide | 0.2 | 38 | |
| Example 11 | HAFT No. 8 of the present invention | 0.4 | | Oxygen absorption test ⑥ |
| | Irganox 1076 | 0.2 | 36 | 30 hours |
| Example 12 | HAFT No. 8 of the present invention | 0.8 | | Oxygen absorption test ⑥ |
| | titanium oxide | 0.05 | 40 | 34 hours |
| | Irganox 1076 | 0.2 | | |
| Comparative Example 7 | none | - | 35 | |
| Comparative Example 8 | Irganox 1076 | 0.2 | 12 | |
| Comparative Example 9 | titanium oxide | 0.2 | | |
| | Irganox 1076 | 0.5 | 18 | |
| Comparative Example 10 | 2,4,6-tris(3,5-di-t-butyl-4-oxy-phenoxy)-S-triazine | 0.4 | 15 | |

Table-3 (continued)

| Examples | Formulations | Amount (phr) | Photo-deterioration test (hr) ⑤ | Notes |
|---|---|---|---|---|
| Comparative Example 11 | 2,6-bis(2,4-hydroxyoxyphenyl)-4-phenyl-S-triazine | 0.4 | 12 | whitening |
| Comparative Example 12 | 1,3,5-tris(di-t-butyl-4-oxybenzyl)-S-triazine-2,4,6-(1H,2H,3H)trion | 0.4 | 10 | |
| Comparative Example 13 | 2,6-bis(3,5-di-t-butyl-4-oxyphenoxy)-4-(n-octylthio)-S-triazine | 0.4 | 18 | |
| Comparative Example 14 | 1,3,5-tris(p-aminobenzoic acid)-ethyl ester | 0.4 | 21 | |
| Comparative Example 15 | 2,6-dimethoxy-4-(2'-hydroxyphenoxy)-S-triazine | 0.4 | 16.5 | |
| Comparative Example 16 | 2,6-tri(octylthio)-4-(4-oxy-3,5-di-t-butylanilino)-S-triazine | 0.4 | 20 | |

As shown in Table-3, addition of the commercially available aging inhibitor to the HAFT compound No. 8 of the present invention (Example 11), addition of the commercially available ultraviolet light shielding agent thereto (Example 10) and addition of the above aging inhibitor and the above ultraviolet light shielding agent thereto (Example 12) resulted in improving light resistance and weather resistance due to their synergistic effect. The above results show that the product of the present invention has advantages over

those containing the conventional ultraviolet light absorbing agent as in Comparative Examples.

Example 5

To ethylene-vinyl acetate (EVA) were added aging inhibitors shown in Table-4 to be subjected to three ply injection blow molding and to prepare a modified polypropylene vial formed by adhering a mixed resin of polyethylene and the above EVA to an intermediate layer.

Respective vials had a transparency of 88%, which was determined by measuring an amount of transmitted light in the wave length range of 590 - 610 nm by use of photoelectric spectrophotometer, a vial wall thickness of 0.25 mm (the thickness of EVA layer: about 0.12 mm), and contained vitamin $K_1$ (marketed by Eizai Co., Ltd., encapsulated product in brown ampul) or vitamin $B_2$ (qualified product in accordance with 10th Revisionary Japanese Pharmacopoeia marketed by Toa Eiyo Kagaku Kogyo Co., Ltd.).

Light resistance test:

Irradiation (about 1000 lux) was carried out from a distance of about 30 cm above a rotary plate, on which samples are placed, by use of two fluorescent chemical lamps (marketed by Tokyo Shibaura Electric Co., Ltd., FL20SBL, major wave length: about 357 nm) and two fluorescent lamps for use in copying (marketed by Matsushita Electric Industrial Co., Ltd., major wave length: about 370 nm).

Determination of vitamin $K_1$:

The absorption spectrum has a maximum wave length of about 327 nm and a minimum wave length of about 285 nm. Measurements were made at 254 nm in a UV tester by use of a high speed liquid chromatography (marketed by Shimadzu Corporation). Permaphase ODS was used as a column. A stainless column having a length of 50 cm was inserted half into the vial under the conditions of mobile phase dioxane : water = 68 : 32 and a flow rate of 0.2 ml/min., followed by declining at an angle of 45°. An irradiated initial concentration of a chemical was taken as 100%. Concentrations (%) of chemicals remaining after the completion of irradiation are shown in Table-4.

Determination of vitamin $B_2$:

It is in the form of a yellow needle-like crystal, and its aqueous solution has a maximum absorption at a wave length of 536 nm and generates fluorescence. Its mixture with sugars may result in photo-decomposition, its mixture with ascorbic acid, phenolic aging inhibitor may result in forming a photo-deterioration inhibitor. It is stable in neutral and acidic states, but is very unstable in an alkaline state. Its determination was carried out by measuring an absorbance at an absorption spectrum of 445 nm by use of a self-recording spectrophotometer (Hitachi EPS-3T type). The initial content of the chemical was taken as 100%. Contents (%) of chemicals remaining after the completion of irradiation are shown in Table-4.

Table-4

| Examples | HAFT compounds of the present invention and their amounts | Notes | Irradiation time (hr) | Amounts of remaining vitamin $K_1$ (%) | Amounts of remaining vitamin $K_2$ (%) |
|---|---|---|---|---|---|
| Example 13 | No. 13 1.0 phr | | 48 | 62 | 78 |
| Example 14 | No. 9 1.0 | | 48 | 66 | 72 |
| Example 15 | No. 9 2.0 | | 7.2 | 82 | 91 |
| Example 16 | No. 14 1.0 | | 48 | 56 | - |
| Example 17 | No. 15 1.0 | | 48 | 54 | - |
| Example 18 | No. 13 1.0 | brown bottle | 72 | 95 | 97 |
| Example 19 | No. 13 1.0 | 1.0 | 48 | 88 | 87 |
| Example 20 | No. 13 1.0 | 1.0 | 48 | 86 | 81 |
| Comparative Example 17 | | brown bottle | 48 | 82 | - |
| Comparative Example 18 | | 1.0 | 48 | 40 | - |
| Comparative Example 19 | | 1.0 | 48 | 58 | - |
| Comparative Example 20 | none | none | 48 | 5 | - |

Note a) Brown glass vial: Transparency determined in the same manner as above: 45%

Note b) Tinuvin P (trade name, marketed by Ciba Geigy A.G., melting point: 129-130°C, $\lambda_{max}$: 298-340 nm)

Note c) Sanol LS-770 (trade name, marketed by Sankyo Co., Ltd., melting point: 81-86°C)

It is found from the results shown in Table-4 that the use of a container formed from a resin composition containing the HAFT as the photo-deterioration inhibitor in the present invention and the preservation of chemicals in the vial as the container make it possible to inhibit the photo-deterioration of the chemicals contained therein, even such chemicals as to be very easily photo-deteriorated, for example, vitamin $K_1$ and vitamin $B_1$ due to its light shielding effect.

Example 6

Into a three-necked flask is charged 500 g of isotactic polypropylene free of volatile matter, followed by melting in a nitrogen stream, while slowly adding 20 g of maleic anhydride, dropping a solution prepared by dissolving 2 g of di-t-butylperoxide in 10 ml of heptane from a dropping funnel into the flask over 30 minutes, reacting at 180°C for about one hour, and distilling off the unreacted maleic anhydride under reduced pressure. To the gelatinized reaction product was added 5 ℓ of carbon tetrachloride, followed by heating at about 110°C under pressure for dissolving it, and distilling off carbon tetrachloride by evaporator to obtain a maleic anhydride-modified polypropylene.

| | | |
|---|---|---|
| (a) | A 20 wt% solution prepared by dissolving the above modified polypropylene in toluene | 500 g |
| (b) | Epikote 828 (epoxy resin having an epoxy equivalent of 184-194 as a condensate between bisphenol A and epichlorohydrin, marketed by Shell Chemical Co., Ltd.) | 4 g |
| (c) | A 10 wt% toluene solution of V-cat-SA-No 102 (amine reaction promoter, marketed by Sun Abot Co., Ltd.) | 0.5 g |
| (d) | Titanium oxide (rutile type, special fine particles 1/20) | 4 g |
| (e) | Compound No. 12 as the HAFT compound of the present invention | 4 g |
| (f) | Sanol LS-772 (trade name, marketed by Sankyo Co., Ltd.) | 1 g |
| (g) | Irganox 1010 (trade name, marketed by Ciba Geigy A.G.) | 1 g |

A mixture of the above (a) - (g) was dispersed for about one hour in a sand mill, followed by properly diluting with toluene, spray coating onto an aluminum sheet, naturally drying for about 10 minutes, and force-drying at about 80°C to obtain a coating having a plate-like surface. The resulting coated film was subjected to 2000 hours' photo-deterioration test and weather resistance test by use of Sunshine Weather Meter, in which a black panel temperature is 60±1°C, a temperature in wet is 40°C, the procedures of irradiation-wetting were repeated at four intervals, with the result that no changes in surface glass, coloring or discoloration, etc.

The present invention provides the following effects.

Addition of hydroxyaryl phenyl triazine compound (HAFT) of the present invention to olefin resin composition provides the resin with highly improved photo-deterioration inhibiting effect.

The container formed from the HAFT-containing resin composition of the present invention makes it possible to inhibit photo-deterioration of the contents contained therein, for example, chemicals, foods, cosmetics, etc., too.

Coating of a formulation thereof with olefin resin composition also makes it possible to provide the above effects.

## Claims

1. A photo-deterioration inhibitor comprising a compound represented by the general formula (I):

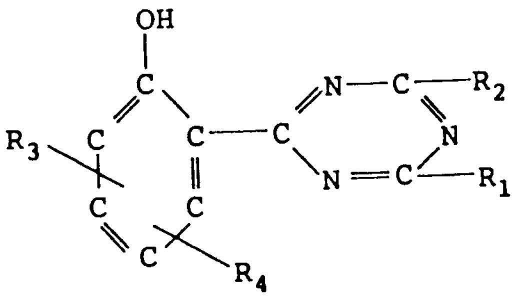

(I)

where $R_1$ and $R_2$ may be the same or different and represent hydrogen, chlorine, hydroxyl group, alkoxy group having 1-18 carbon atoms, aryloxy group having 6-8 carbon atoms and mono- or dialkylamino group having 1-4 carbon atoms, respectively, and $R_3$ and $R_4$ may be the same or different and represent hydrogen, chlorine, alkyl group having 1-8 carbon atoms, methoxy group and ethoxy group.

2. A photo-deterioration inhibitory resin composition comprising a compound represented by the general formula (I) as defined in claim 1 together with a polyolefin resin or polyolefin resin composition.

3. A photo-deterioration inhibitor or photo-deterioration inhibitory resin as claimed in claim 1 or claim 2 which further comprises an aging inhibitor, an ultraviolet light absorbing agent, an ultraviolet light shielding agent or two or more thereof.

4. A container formed from a photo-deterioration inhibitory resin composition as claimed in claim 2 or claim 3.

5. A method for preparing a photo-deterioration inhibitory resin which comprises mixing a compound represented by the general formula (I) as defined in claim 1 with a polyolefin resin or polyolefin resin composition.

6. A method as claimed in claim 5 in which an aging inhibitor, an ultraviolet light absorbing agent, an ultraviolet light shielding agent or two or more thereof is also mixed with the compound of formula (I) and a polyolefin resin or a polyolefin resin composition.

7. A method of making a container which comprises mixing a compound represented by the general formula (I) as defined in claim 1 with a polyolefin resin or polyolefin resin composition, melting the mixture by heating and thereafter molding the mixture to form the shape of the container.

8. A method as claimed in claim 7 wherein other additives are included in the mixture prior to melting.

9. A method as claimed in claim 7 or claim 8 in which the molding is effected by injection molding or T blow molding.

# FIG. 1

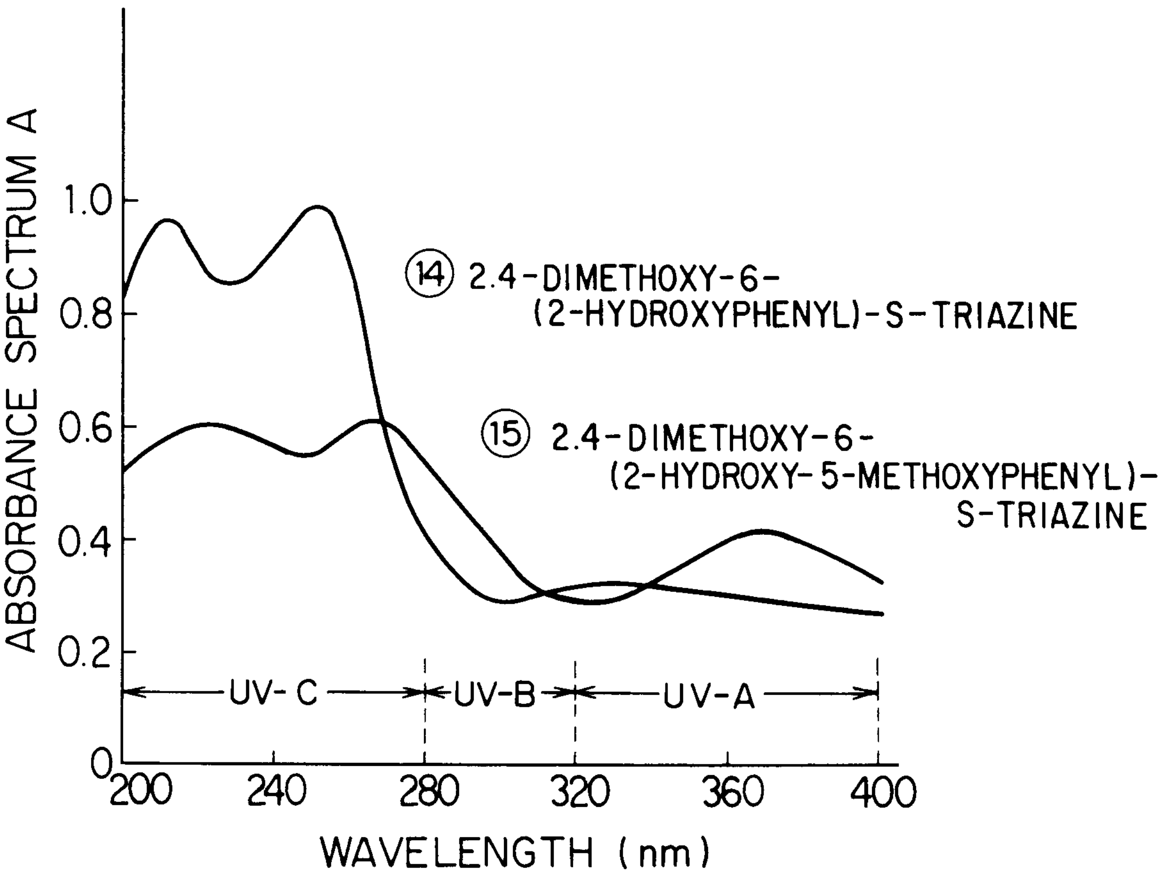
ABSORBANCE SPECTRUM A
1.0
0.8
0.6
0.4
0.2
0
200
240
280
320
360
400
WAVELENGTH (nm)
UV-C
UV-B
UV-A
⑭ 2.4-DIMETHOXY-6-(2-HYDROXYPHENYL)-S-TRIAZINE
⑮ 2.4-DIMETHOXY-6-(2-HYDROXY-5-METHOXYPHENYL)-S-TRIAZINE

European Patent Office

# EUROPEAN SEARCH REPORT

Application Number

EP 91 30 7271

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | GB-A-1 142 295 (CIBA LIMITED)<br>* Formulas 5c,9,22,24,43,70,87 *<br>* claims 1,6,14-19; examples 2,7,10-14,17 *<br>--- | 1-2,4-5 | C08K5/3492<br>C08L23/02<br>C08J3/20<br>C07D251/14<br>B65D1/00 |
| X | GB-A-1 126 979 (CIBA LIMITED)<br>* Formulas 57 and 93 *<br>* page 16, line 22 - page 19, line 5; claims 1-2,6,7,12-13; examples 11-14 *<br>--- | 1-2,4-5 | |
| A | GB-A-1 011 575 (J.R. GEIGY AG)<br>* column 6, line 3 - column 8, line 48; claim 1 *<br>----- | 1-3,5-6 | |
| | | | TECHNICAL FIELDS SEARCHED (Int. Cl.5 ) |
| | | | C08K<br>C08L<br>C07D |

The present search report has been drawn up for all claims

| Place of search | Date of completion of the search | Examiner |
|---|---|---|
| THE HAGUE | 18 MARCH 1992 | FUHR C.K.B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)